# EUROPÄISCHE PATENTSCHRIFT

(11) **EP 1 954 664 B1**
(45) Veröffentlichungstag und Bekanntmachung des Hinweises auf die Patenterteilung: **07.10.2015**
(21) Anmeldenummer: 06830066.4
(22) Anmeldetag: 21.11.2006
(51) Int. Cl.: B01J 19/00, C07C 51/215, C07C 51/25

(54) **VERFAHREN ZUM SICHEREN BETREIBEN EINER GASPHASEN-PARTIALOXIDATION**
METHOD FOR SAFELY CARRYING OUT GAS PHASE PARTIAL OXIDATION
PROCÉDÉ POUR LE FONCTIONNEMENT SÛR D'UNE OXYDATION PARTIELLE EN PHASE GAZEUSE

(30) Priorität: 23.11.2005 DE 102005055826; 23.11.2005 US 738994 P
(43) Veröffentlichungstag der Anmeldung: 13.08.2008
(73) Patentinhaber: BASF SE, 67056 Ludwigshafen (DE)
(72) Erfinder: HECHLER, Claus, 67063 Ludwigshafen (DE); SCHLEMMER, Peter, 67304 Eisenberg (DE)
(86) Internationale Anmeldenummer: PCT/EP2006/068718
(87) Internationale Veröffentlichungsnummer: WO 2007/060163

(56) Entgegenhaltungen:
- WO-A-2004/007405
- US-A1- 2004 181 090

## Beschreibung

Die Erfindung betrifft ein Verfahren zum sicheren Betreiben einer kontinuierlich betriebenen heterogen katalysierten Gasphasen-Partialoxidation wenigstens einer organischen Ausgangsverbindung in einem Reaktor, dessen Beschickungsgasgemischstrom neben der wenigstens einen partiell zu oxidierenden organischen Ausgangsverbindung und molekularem Sauerstoff als Oxidationsmittel wenigstens ein sich unter den Bedingungen der heterogen katalysierten Gasphasen-Partialoxidation im Wesentlichen inert verhaltendes Verdünnungsgas umfasst und der durch Zusammenführen wenigstens zweier verschiedener Ausgangsströme erzeugt wird, bei dem unter Einbezug der online-Messung der Konzentration eines oder mehrerer ausgewählter Bestandteile im Beschickungsgasgemischstrom selbst, in einem oder mehreren der den Beschickungsgasgemischstrom erzeugenden Ausgangsströme und/oder im Produktgasgemischstrom verhindert wird, dass der Reaktor mit einem explosionstechnisch oder anderweitig nicht mehr beherrschbaren Beschickungsgasgemischstrom beschickt wird, wobei zur online-Messung ein Teilstrom des jeweils zu analysierenden Gasstroms kontinuierlich (auf einer Seite) in eine Messzelle eines Analysengerätes hinein- und während der durchgeführten Messung (auf einer anderen Seite) aus der Messzelle des Analysengeräts in eine Entlassatmosphäre herausgeführt wird.

Unter der Partialoxidation einer organischen Ausgangsverbindung mit molekularem Sauerstoff als Oxidationsmittel werden diejenigen Umsetzungen der organischen Ausgangsverbindung im Beisein von molekularem Sauerstoff zu wenigstens einer von CO, CO₂ und H₂O verschiedenen Zielverbindung verstanden, bei denen die Oxidationszahl wenigstens eines C-Atoms in der wenigstens einen organischen Zielverbindung größer ist als in der organischen Ausgangsverbindung. Partialoxidationen sind damit insbesondere alle Umsetzungen einer organischen Ausgangsverbindung mit molekularem Sauerstoff zu wenigstens einer von CO, CO₂ und H₂O verschiedenen, wenigstens ein Sauerstoffatom mehr als die organische Ausgangsverbindung enthaltenden Zielverbindung. Im Besonderen sind auch Oxydehydrierungen und oxidative Dehydrierungen (bei letzteren wird wenigstens intermediär molekulare Wasserstoff gebildet und selbiger in einem Folgeschritt wenigstens teilweise zu H₂O oxidiert) von gesättigten Kohlenwasserstoffen zu ungesättigten Kohlenwasserstoffen Partialoxidationen (z.B. von Propan zu Propylen).

Partialoxidationen organischer Ausgangsverbindungen werden in großem Umfang zur Herstellung organischer Zielverbindungen unterschiedlichster Art eingesetzt (z.B. die Herstellung von Acrylsäure aus Propen und/oder Propan, die Herstellung von Ethylenoxid aus Ethylen, die Herstellung von Methacrylsäure aus Isobuten und/oder Isobutan, oder die Herstellung von Propylen aus Propan). Es werden hierzu normalerweise wenigstens zwei stoffliche Ausgangsströme, nämlich wenigstens ein die wenigstens eine organische Ausgangsverbindung (die Reaktionsausgangssubstanz bzw. -substanzen) enthaltender und wenigstens ein molekularen Sauerstoff enthaltender Ausgangsstrom zum Beschickungsgasgemischstrom miteinander gemischt, wobei die Mischung in der Lage ist, unter geeigneter Aktivierung (z.B. durch einen Katalysator) in erwünschter Weise zu einem die wenigstens eine Zielverbindung enthaltenden Produktgasgemischstrom zu reagieren. Die hierbei zu verwendenden chemischen Reaktionsausgangssubstanzen (organischen Ausgangsverbindungen) haben in der überwiegenden Anzahl aber die Eigenschaft, in Anwesenheit von molekularem Sauerstoff neben der erwünschten Reaktion zur Zielverbindung in gewissen Zusammensetzungsbereichen des Beschickungsgasgemischs auch unerwünscht abreagieren zu können, wobei dieser Reaktionsverlauf in der Regel durch eine nicht gezielt eingesetzte (d.h., normalerweise unbeabsichtigt vorhandene) Ursache ausgelöst (z.B. eine ungewollte Zündquelle wie einen Funken oder eine unbeabsichtigt überhitzte Oberfläche) wird und sich dann mit erheblichem Druckanstieg deflagrativ als Explosion oder sogar als Detonation entwickeln kann.

Eine sichere Beherrschung der Auswirkungen aller möglichen unerwünschten Reaktionsabläufe wäre, wenn überhaupt, nur mit enormem Kostenaufwand zu leisten, beispielsweise durch eine überaus teure Ausführung der Reaktionsapparate, die dann für weit höheren Druck als er im Normalbetrieb auftreten kann, dimensioniert sein müssen, weshalb in der sicheren Vermeidung bestimmter gefährlich explosiver Beschickungsgasgemischsammensetzungen im vorhinein zur Verhinderung bestimmter unerwünschter Reaktionsabläufe ein großes wirtschaftliches Potential begründet liegt. Da sich im Verlauf einer Partialoxidation die Reaktanden verbrauchen, sind die im Verlauf einer Partialoxidation aus dem Beschickungsgasgemisch erwachsenden Reaktionsgasgemische in der Regel immer dann gut beherrschbar, wenn das Beschickungsgasgemisch selbst gut beherrschbar ist.

Eine grundlegende Übersichtsbeschreibung, wie auch eine dem Stand der Technik und den Regularien angemessene Vorgehensweise und Absicherung ist in dem Leitfaden zur Richtlinie 1999/92/EG der EU-Kommission in der Endfassung vom April 2003 zu entnehmen.

Eine generelle Vermeidung zündfähiger Beschickungsgasgemische ist dadurch erreichbar, dass der Gehalt der organischen Anteile im Beschickungsgasgemisch sicher unter dem für eine Zündfähigkeit erforderlichen Mindestwert (untere Explosionsgrenze; durch Mitverwendung inerter Verdünnungsgase (das sind Gase, die im Verlauf der Partialoxidation zu wenigstens 95 mol-%, bevorzugt zu wenigstens 98 mol-% und mehr unverändert erhalten bleiben; typische inerte Verdünnungsgase sind z.B. molekularer Stickstoff, Edelgase und Wasserdampf; inertes Verdünnungsgas kann aber auch ein selbst zündbares Gas wie z.B. Propan im Fall einer Partialoxidation von Propylen zu Propylenoxid sein) lässt sich der Verlauf von Explosionsgrenzen beeinflussen) gehalten wird. Konsequenz einer solchen Vorgehensweise sind beim Einsatz von den vorgenannten Gehalt kontrollierenden Messeinrichtungen nach dem Stand der Technik in der Regel derart gering konzentrierte Beschickungsgasgemische (sehr weit unterhalb der möglichen Grenzkonzentration liegende), dass die angestrebte Reaktion wirtschaftlich nicht vorteilhaft oder nicht sinnvoll möglich ist.

Eine alternative Möglichkeit zur Vermeidung zündfähiger Beschickungsgasgemische besteht in der sicheren Erhöhung der Konzentration der organischen Anteile über einen Maximalwert, die sogenannte obere Explosionsgrenze, hinaus. Nachteilig an einer solchen Vorgehensweise ist, dass beim Einsatz von diese Konzentration kontrollierenden Messeinrichtungen nach dem Stand der Technik die dabei resultierenden Beschickungsgasgemische mit Blick auf die Zielreaktion in der Regel (sehr weit oberhalb dieser Obergrenze liegende) Zusammensetzungen aufweisen, die einerseits nur wenig reaktiv sind, so dass nur geringe Reaktionsraten resultieren, und andererseits meist einen sehr hohen Überschuss der organischen Reaktionsausgangssubstanz aufweisen, aus dem die Notwendigkeit erwächst, diesen Überschuss aus dem resultierenden Produktgasgemisch abtrennen und ins Beschickungsgasgemisch rückführen zu müssen, was beides wirtschaftlich von Nachteil ist. Auch wirkt sich ein Überschuss der Konzentration der organischen Anteile relativ zur Reaktionsstöchiometrie normalerweise negativ auf die Katalysatorstandzeit aus.

Eine dritte Möglichkeit besteht z.B. darin, die Konzentration an molekularem Sauerstoff im Beschickungsgasgemisch unter einen unteren Grenzwert, die sogenannte Sauerstoffgrenzkonzentration, abzusenken. Unabhängig von der sonstigen Zusammensetzung des Beschickungsgasgemisches sind dann keine Zündungen mehr möglich. Beim Einsatz von die Sauerstoffkonzentration kontrollierenden Messeinrichtungen nach dem Stand der Technik erfordert dies eine signifikante Unterschreitung der vorgenannten Grenzkonzentration, als deren Folge in der Regel ebenfalls keine hohen Reaktions- und Umsatzraten möglich sind.

Um diese Nachteile zu vermeiden, bedarf es die Gehalte bzw. Konzentrationen kontrollierender Messeinrichtungen, die auch im Regelbetrieb eine möglichst weitgehende Ausnutzung des noch sicheren Zusammensetzungsbereichs im Sinne einer möglichst weitgehenden aber noch sicheren Annäherung an die Grenzen zündfähiger Beschickungsgasgemischzusammensetzungen gewährleisten, und die durch die daraus resultierenden hohen Partialdrücke der Reaktanden die höchstmöglichen Umsetzungsdichten und Umsetzungsraten gerade auch für den gewünschten Reaktionsablauf der Partialoxidation und somit in aller Regel die wirtschaftlich vorteilhafteste Betriebsweise ermöglichen.

Bei verschiedenen heterogen katalysierten partiellen Gasphasen-Oxidationen (beispielsweise bei der Partialoxidation von Xylolen zu Phthalsäureanhydrid) ist es bezüglich des reaktionstechnischen Ablaufs und einer ökonomisch sinnvollen Betriebsweise sogar erstrebenswert, Beschickungsgasgemischzusammensetzungen einzustellen, die zwar innerhalb des Bereichs zündfähiger Zusammensetzungen liegen, deren Verhalten im Fall einer unerwünschten Zündung jedoch durch geeignete anlagentechnische Ausstattung und/oder Auslegung noch mit vergleichsweise begrenztem Aufwand sicher beherrschbar ist. Ein volles Ausschöpfen der vorgenannten Option erfordert jedoch Gehalte/Konzentrationen kontrollierende Messeinrichtungen, die eine sicher begrenzte, d.h., eine genau kontrollierte Überschreitung der Zündgebietsgrenzen gewährleisten, und ein unzulässig weites Vordringen in zündfähiges Beschickungsgasregime mit gegebenenfalls nicht mehr kontrollierbaren Reaktionsabläufen ausschließen.

Die kontrolliert begrenzte Überschreitung der Grenzen des den organischen Bestandteilen eines Beschickungsgasgemischs zuordenbaren Zündgebiets und das gleichzeitige Einhalten eines ausreichend sicheren Abstands der Beschickungsgasgemischzusammensetzung von z.B. zündfähigen stöchiometrischen Mischungen, d.h. solchen Mischungen, bei denen die molaren Verhältnisse von reaktionsfähigem Sauerstoff und organischen Bestandteilen für eine Vollverbrennung des Kohlenstoffs der organischen Bestandteile zu CO₂ stöchiometrisch ideal passend sind, und bei denen in Folge dessen die Auswirkungen unerwünschter Zündungen in Form von z.B. drastischem Druck- und Temperaturanstieg am stärksten, das heißt am ungünstigsten und am wenigsten beherrschbar wären, eröffnet vielfach überhaupt erst die Möglichkeit, gewisse partialoxidative Herstellverfahren wirtschaftlich sinnvoll durchführen zu können.

Es ist daher von hohem Interesse und Vorteil, es durch geeignete Kontroll- und Begrenzungsmaßnahmen sicher zu ermöglichen, sich den Grenzen zündfähiger Beschickungsgaszusammensetzungen entweder soweit wie möglich anzunähern ohne diese Grenzen zu überschreiten, oder aber diese Grenzen gerade soweit zu überschreiten, als die Auswirkungen einer unplanmäßigen Abreaktion im Fall einer solchen Überschreitung apparativ noch kostenvorteilhaft beherrschbar sind. Grundlage derartiger Kontroll- und Begrenzungsmaßnahmen ist eine möglichst genaue Kenntnis der verschiedenen Prozessparameter und hier insbesondere der Konzentrationen der vorhandenen relevanten Bestandteile des Beschickungsgasgemischs, wie z.B. in der Schrift DE- A 102 32 482 ausgeführt wird. Um diese Kenntnis zu erlangen, ist es üblich, die Konzentrationen von ausgewählten Bestandteilen im Beschickungsgasgemischstrom selbst, in einem oder mehreren der den Beschickungsgasgemischstrom erzeugenden Ausgangsströme und/oder im durch die Gasphasen-Partialoxidation erzeugten Produkt-gasgemischstrom (z.B. vom im Produktgasgemischstrom noch enthaltenen, in der Partialoxidation nicht umgesetzten molekularen Restsauerstoff) experimentell online zu bestimmen, die erhaltenen Resultate mit der Erzeugung des Beschickungsgasgemischstroms rückzukoppeln und im Extremfall die Partialoxidation durch Vorenthaltung von organischer Ausgangssubstanz abzubrechen (vgl. DE-A 102 32 482).

Ein nicht mehr beherrschbares Beschickungsgasgemisch kann aber auch dann vorliegen, wenn der Gehalt des Beschickungsgasgemischs an molekularem Sauerstoff insofern zu gering ist, als dass die in der Regel Multielementoxidmassen enthaltenden, in ihrer Herstellung normalerweise äußerst kostspieligen Katalysatoren während der Partialoxidation nicht mehr in ausreichendem Umfang reoxidiert und dadurch gegebenenfalls irreversibel geschädigt werden, was in letzter Konsequenz gleichfalls eine Unterbrechung der Partialoxidation nach sich zieht. Im Übrigen kann der Produktgasgemischstrom nach Zielproduktabtrennung aus selbigem in partieller oder in vollständiger Kreisgasfahrweise auch selbst Ausgangsstrom zur Erzeugung des Beschickungsgasgemischstromes sein.

In US-A 2004/0181090 wird beschrieben, dass die Messeinrichtungen bzw. Systeme des Standes der Technik zur Ermittlung solcher Gaskonzentrationen erhebliche Ungenauigkeiten aufweisen und daher nicht ausreichend vertrauenswürdig sind. Als Lösungsansatz wird als Alternative zu erhöhten Sicherheitsabständen von den zulässigen Grenzzusammensetzungen vorgeschlagen, erst bei gleichzeitiger Anzeige des unzulässigen Zustandes durch eine zweite Vergleichs- oder Kontrollmethode das Vorhandensein eines Fehlers anzunehmen und eine Abschaltung der Gasphasen-Partialoxidation herbeizuführen. Dies führt jedoch in wenig befriedigender Weise zu einem annähernd doppelt so hohen Installationsaufwand mit entsprechend unwirtschaftlichen Kosten, wobei zumindest für einen Teil der Einrichtungen eine fragwürdige Zuverlässigkeit in Kauf genommen wird.

Eigene Untersuchungen der möglichen Ursachen für vorgenannte Ungenauigkeiten haben folgendes ergeben.

Die überwiegende Anzahl bekannter Messverfahren zur online Bestimmung von Konzentrationen in Gasgemischen ermitteln eine Teilchendichte (Anzahl von Teilchen je Volumeneinheit) der zu bestimmenden Spezies in einem vorgegebenen Gasvolumen (z.B. das Volumen einer Messzelle). Die Anzahl der Teilchen, z.B. Moleküle, im Messvolumen gehorcht den anerkannten physikalischen Gesetzmäßigkeiten auf dem Gebiet der phänomenologischen Thermodynamik und ist im Bereich der Gültigkeit des idealen Gasgesetzes, die typischerweise für solche Messungen angenommen werden kann, direkt proportional dem Absolutdruck und umgekehrt proportional der Absoluttemperatur im Messvolumen.

Beispielsweise kann die Konzentrationsbestimmung (insbesondere im Fall von molekularem Sauerstoff) nach den in den Schriften DE-A 10117678 und DE-A 102005052923 beschriebenen Verfahrensweisen erfolgen. Diese beruhen unter anderem darauf, dass elektromagnetische Strahlung mit einer Wellenlänge, bei der die zu bestimmende Spezies absorbiert, durch die Messzelle des Analysengerätes geleitet und der nicht von der in der Messzelle befindlichen zu bestimmende Spezies absorbierte Anteil der elektromagnetischen Strahlung hinter der Messzelle gemessen wird.

Beispielsweise kann die Sauerstoffkonzentration mittels eines Laserstrahls gemessen werden, dessen Wellenlänge auf eine der Rotations-Feinstrukturbanden von molekularem Sauerstoff eingestellt ist. Zum Eichen der Messung kann der Laserstrahl eine Kalibrierzelle durchstrahlen, die ein Gas mit definiertem Sauerstoffgehalt enthält oder durch die ein Gas mit definiertem Sauerstoffgehalt geleitet wird. Beispielsweise kann der vorgenannte Laser ein Diodenlaser sein, dessen Wellenlänge auf eine der Rotations-Feinstruktur-Banden von molekularem Sauerstoff im Bereich von 759,5 bis 768 nm einstellbar ist. Der Modulationsbereich kann dabei + 0,05 nm betragen. Grundsätzlich kann für den erfindungsgemäßen Zweck z.B. ein Gasanalysengerät vom Typ Ultramat^{R} 23 der Firma Siemens eingesetzt werden (arbeitet im IR-Bereich). Vorgenanntes Messverfahren eignet sich insbesondere zur simultanen Bestimmung von Propan und/oder Propylen im Infrarotbereich (IR). Dazu können eignen sich unter anderem Messgeräte vom Typ MCS 100 (Multifrequenzgerät) oder vom Typ Unor^{R}, jeweils der Firma Sick-Maihak in DE- Reute.

Für das erfindungsgemäße Verfahren eignen sich aber auch Messverfahren, die auf völlig anderen Zusammenhängen basieren. Beispielsweise weist molekularer Sauerstoff eine vergleichsweise große paramagnetische Suszeptibilität auf. Dies wird von Sauerstoff-Analysatoren der Serie OxymatR der Firma Siemens sowie von Sauerstoff-Analysatoren der Serie PMA^{R} der Fa. M&C Products Analysentechnik GmbH in D-40885 Ratingen ausgenützt. Hierbei handelt es sich um Analysengeräte, die aufgrund ihrer sehr schnellen Ansprechzeit, ihres geringen Totvolumens, ihrer geringen Querempfindlichkeit gegenüber anderen Gasbestandteilen sowie der direkten Durchströmbarkeit der Messzelle besonders vorteilhaft sind (z.B. die Version PMA 30). Die Messverfahren auf der Grundlage des vorgenannten Prinzips gehören zu den genauesten quantitativen Bestimmungsverfahren für molekularen Sauerstoff im Bereich von 0 bis 100 Vol-%. An Spannbändern ist bei den vorgenannten Geräten eine diamagnetische Hantel mit im Drehpunkt befindlichem Spiegel befestigt und in einem inhomogenen Magnetfeld montiert. Der Sauerstoff strebt infolge seines Paramagnetismus in das inhomogene Magnetfeld der Messzelle. Die O₂-Moleküle üben dabei auf die Hantel ein Drehmoment aus und lenken sie entsprechend der Sauerstoffkonzentration aus. Durch optische Abtastung wird elektronisch ein Strom erzeugt, der durch eine Drahtschleife fließt, die um die Hantel gelegt ist und diese in die neutrale Lage zurück dreht. Der Kompensationsstrom ist proportional zum Sauerstoffgehalt des Messgases, wodurch die O₂-Anzeige absolut linear ist.

Grundsätzlich kommen prinzipiell aber auch gaschromatographische oder elektrochemische Verfahren in Betracht. Bei letzteren wird die zu analysierende Spezies durch geeignete Energiezufuhr ionisiert und kontinuierlich die elektrische Leitfähigkeit des in der Messzelle befindlichen Gases bestimmt.

Nach den Verfahren des Standes der Technik wird zum Zweck der Durchführung einer online Analyse eines Gasstroms diesem Gasstrom normalerweise über eine geeignete Mengendosiereinrichtung ein Teilstrom gleicher Zusammensetzung entnommen, der dann entweder aufgrund des Gefälles zwischen dem Druck im zu analysierenden Gasstrom und dem Druck im Messvolumen (in der Messzelle des Messgeräts) und/oder durch eine Fördereinrichtung, welche ein solches Druckgefälle erzeugt, in das Messvolumen (in die Messzelle) des Messgeräts und von dort wiederum aufgrund des Druckgefälles zu einer Entlassatmosphäre (das ist die Atmosphäre, in die der Teilstrom nach durchgeführter Messung entlassen wird) geführt wird; in einfachster Weise kann dies die Umgebungsatmosphäre sein; die Entlassatmosphäre kann aber auch eine künstlich erzeugte Atmosphäre sein, z.B. die Atmosphäre eines mittels Vakuumpumpen unter reduziertem Druck (oder unter überatmosphärischem Druck) befindlichen Raums) in dieselbe strömt, wobei sich innerhalb des Messvolumens (innerhalb der Messzelle) aufgrund des sehr niedrigen Druckabfalls in der Wegströmleitung regelmäßig praktisch der gleiche Druck wie derjenige der Entlassatmosphäre aber insbesondere die Schwankungen der Entlassatmosphäre einstellen.

In entsprechender Weise bilden sich in der Messzelle auch Druckschwankungen im zu analysierenden Gasstrom ab.

Änderungen und/oder Schwankungen von Druck und/oder Temperatur im Messvolumen (der Messzelle) führen aber unmittelbar zu veränderten Werten der zu ermittelnden Konzentration und sind vermutlich ursächlich für die in der US-A 2004/0181090 angesprochenen Probleme. Zusätzlich bedingen sie wegen der Notwendigkeit, zu den oben beschriebenen, zu vermeidenden zündfähigen Zusammensetzungen sicher festgestellte Mindestabstände zu halten, eine Einschränkung des zugänglichen Konzentrationsbereichs für die gewünschte partielle Oxidation, da den diskutierten Ungenauigkeiten durch einen erhöhten Sicherheitsabstand Rechnung getragen werden muss. Alternativ ist eine Kontrollvariante gemäß der Lehre der US-A 2004/0181090 erforderlich.

Aufgabe der vorliegenden Erfindung war es daher, die Konzentrationsmessung von insbesondere bezüglich der für die zündtechnische Sicherheit relevanten Substanzen eines Beschickungsgasgemischs für eine Partialoxidation prinzipiell mit einer solchen Genauigkeit zu ermöglichen, dass diese Messung nicht das einschränkende Element bezüglich des sicher nutzbaren Zusammensetzungsbereichs des Beschickungsgases darstellt und gleichzeitig auf eine kostenaufwändige und bezüglich der sicherheitstechnischen Tragfähigkeit eingeschränkte Methodik, wie in US 2004/0181090 beschrieben, verzichtet werden kann.

Die Lösung besteht in einem Verfahren zum sicheren Betreiben einer kontinuierlich betriebenen heterogen katalysierten Gasphasen-Partialoxidation wenigstens einer organischen Ausgangsverbindung in einem Reaktor, dessen Beschickungsgasgemischstrom neben der wenigstens einen partiell zu oxidierenden organischen Ausgangsverbindung und molekularem Sauerstoff als Oxidationsmittel wenigstens ein sich unter den Bedingungen der heterogen katalysierten Gasphasen-Partialoxidation im Wesentlichen inert verhaltendes Verdünnungsgas umfasst und der durch Zusammenführen wenigstens zweier verschiedener Ausgangsströme erzeugt wird, bei dem unter Einbezug der online-Messung der Konzentration eines oder mehrerer ausgewählter Bestandteile im Beschickungsgasgemischstrom selbst, in einem oder mehreren der den Beschickungsgasgemischstrom erzeugenden Ausgangsströme und/oder im Produktgasgemischstrom verhindert wird, dass der Reaktor mit einem explosionstechnisch oder anderweitig nicht mehr beherrschbaren Beschickungsgasgemischstrom beschickt wird, wobei zur online-Messung ein Teilstrom des jeweils zu analysierenden Gasstroms kontinuierlich in eine Messzelle eines Analysengerätes hinein- und während der durchgeführten Messung aus der Messzelle des Analysengeräts in eine Atmosphäre (die "Entlassatmosphäre") herausgeführt (entlassen) wird, wobei der zu analysierende Gasstrom, und/oder die Entlassatmosphäre Druckschwankungen unterliegen, das dadurch gekennzeichnet ist, dass der Einfluss von Schwankungen des Druckes des zu analysierenden Gasstroms und/oder der Entlassatmosphäre auf den Messdruck in der Messzelle im Analysengerät und damit auf das Messergebnis
a) rechnerisch korrigiert und/oder
b) dadurch minimiert wird, dass der Messdruck in der Messzelle des Analysengeräts unabhängig vom Druck des zu analysierenden Gasstroms und/oder der Entlassatmosphäre mittels einer Druckregeleinrichtung konstant gestellt oder geregelt wird.

Die weitaus meisten Messverfahren zur online Bestimmung von Konzentrationen in der Gasphase ermitteln, wie bereits erwähnt, eine Teilchendichte der zu analysierenden Substanz. Die Anzahl der Teilchen, z.B. Moleküle, je Messvolumen gehorcht den anerkannten physikalischen Gesetzmäßigkeiten und ist im Bereich der Gültigkeit des idealen Gasgesetztes direkt proportional zum Absolutdruck im Messvolumen und umgekehrt proportional zur Absoluttemperatur im Messvolumen. Bei Kenntnis von Druck und Temperatur in der Messzelle ist vorgenannte rechnerische Korrektur somit vergleichsweise einfach möglich.

Es wurde ferner gefunden, dass der wesentliche äußere (nicht im eigentlichen Messprinzip angesiedelte) Einflussfaktor einer solchen Messung nach Verfahren gemäß des Standes der Technik insbesondere die Schwankung des Messdruckes in der Messzelle ist, welche durch Druckregelungsmaßnahmen auf ein weitgehend verschwindendes Maß reduziert werden kann, wodurch das aufgrund sicherheitstechnisch begründeter Toleranzen zuvor frühzeitig herbeigeführte Abschalten der betriebenen Gasphasen-Partialoxidation weitgehend vermieden werden kann bzw. seltener und/oder später erfolgt, mit der Konsequenz besserer Wirtschaftlichkeit durch weitest gehende Ausnutzung der sicherheitstechnisch zulässigen Beschickungsgaszusammensetzungsbereiche und durch bessere bzw. intensivere Reaktion aufgrund der höheren Reaktivität des Beschickungsgasgemisches.

Nach den Verfahren des Standes der Technik wird, wie bereits beschrieben, zum Zweck der Durchführung einer online Analyse eines Gasstroms diesem Gasstrom normalerweise über eine geeignete Mengendosiereinrichtung (im einfachsten Fall ein fester oder ein variabel einstellbarer Querschnitt) ein Teilstrom gleicher Zusammensetzung entnommen, der dann entweder aufgrund des Gefälles zwischen dem Druck im zu analysierenden Gasstrom und dem Druck im Messvolumen (in der Messzelle des Messgeräts) und/oder durch eine Fördereinrichtung, welche ein solches Druckgefälle erzeugt, in das Messvolumen (in die Messzelle) des Messgeräts und von dort wiederum aufgrund des Druckgefälles zu einer Entlassatmosphäre (das ist die Atmosphäre, in die der Teilstrom nach durchgeführter Messung entlassen bzw. geführt wird; in einfachster Weise kann dies die Umgebungsatmosphäre sein; die Entlassatmosphäre kann aber auch eine künstlich erzeugte Atmosphäre sein, z.B. die Atmosphäre eines mittels Vakuumpumpen unter reduziertem Druck befindlichen Raums) in dieselbe strömt.

Ein nach dieser Verfahrensweise gemäß Stand der Technik dem Messgerät im Messvolumen (in der Messzelle) aufgeprägter Druck der Entlassatmosphäre kann nun gemäß vorliegender Erfindung in einfacher Weise durch die Zwischenschaltung (zwischen Messzelle und Entlassatmosphäre) einer einstell- und/oder regelbaren Durchströmöffnung vom Druck der Entlassatmosphäre entkoppelt und geregelt werden. Diese Durchströmöffnung wird entsprechend der Druckzustände im Messvolumen (in der Messzelle) und in der Entlassatmosphäre so variiert, dass im Messvolumen (in der Messzelle) ein weitgehend konstanter Druck unabhängig vom Druck in der Entlassatmosphäre und auch unabhängig vom ins Messvolumen (in die Messzelle) strömenden Teilstrom vorherrscht.
D.h., das Grundprinzip der erfindungsgemäßen Druckregelung besteht darin, den durch die Messzelle des Analysengeräts strömenden Teilstrom auf einen von den jeweiligen aktuellen Drücken in der Entlassatmosphäre bzw. im zu analysierenden Gasstrom unabhängigen Wert einzuregeln.

Erfindungsgemäß zweckmäßig wird dieses Grundprinzip mittels einer zwischen Messzelle und Entlassatmosphäre angeordneten Druckregeleinrichtung in Form eines verstellbaren Durchlasses, z.B. in der Ausprägung eines Regelventils mit geeigneter Verstellantriebsvorrichtung (z.B. eine bewegliche Nadel mit konischem Querschnitt) ausgeführt. Als Antriebsenergien kommen sowohl Eigenenergien des Messsystems selbst wie z.B. auf Membranen wirkende Druckdifferenzen (z.B., die zwischen Entlassatmosphäre und Messzelle bestehende Druckdifferenz oder die zwischen Messzelle und zu analysierendem Gasstrom bestehende Druckdifferenz ) aber auch Fremdenergien wie z.B. vom eigentlichen Messsystem abgekoppelte Gas- und Flüssigkeitsdrucke (z.B. "Druckluft") und/oder z.B. mittels Feder(n) aufgebrachte mechanische Energie in Betracht.

Es stellt sich so ein geregeltes Gleichgewicht zwischen aus dem zu analysierenden Gasstrom aus der Gasphasen-Partialoxidation in das Messvolumen (in die Messzelle) zuströmender und aus dem Messvolumen in die Entlassatmosphäre abströmender Gasmenge mit der Wirkung der Beibehaltung eines nahezu konstant eingeregelten Druckes im Messvolumen selbst ein.

Das Einfügen einer weiteren entsprechend gestalteten Druckregeleinrichtung zwischen zu analysierendem Gasstrom und Messzelle verbessert die Druckkonstanz in der Messzelle zusätzlich.

Erfindungsgemäß zweckmäßig wird man zusätzlich zur Anwendung einer erfindungsgemäßen Druckregelung den Einfluss der Schwankungen der Messtemperatur auf das Messergebnis dadurch reduzieren, dass die Temperatur des Messvolumens (der Messzelle) konstant geregelt wird. Hierzu kann das Analysengerät oder können die Analysengeräte beispielsweise in einem klimatisierten Raum aufgestellt werden. Eine Thermostatisierung der Messzelle innerhalb des Analysengerätes ist jedoch häufig ausreichend.

Das erfindungsgemäße Verfahren ist zur online-Messung der Konzentration eines oder mehrerer ausgewählter Bestandteile im Beschickungsgasgemischstrom selbst, im Produktgasgemischstrom und/oder in einem oder mehreren der den Beschickungsgasgemischstrom erzeugenden Ausgangsströme vorgesehen, das heißt zur Konzentrationsmessung im laufenden Betrieb einer kontinuierlichen heterogen katalysierten Gasphasen-Partialoxidation.

Die erfindungsgemäß einzusetzende Druckregeleinrichtung, um den Messdruck in der Messzelle des Analysengeräts unabhängig vom aktuellen Druck der Entlassatmosphäre konstant zu stellen oder zu regeln, kann vorteilhaft als einstückige, kompakte Einheit, die insbesondere handelsüblich sein kann, ausgebildet sein und wird als solche zweckmäßig am Austritt des Gasstroms aus der Messzelle, d.h. zwischen dem Analysengerät und der Entlassatmosphäre installiert.

Es ist auch möglich, die Druckregeleinrichtung als nicht-kompakte Einheit auszubilden, wobei zwei oder mehrere der sie konstituierenden funktionellen Einheiten - Messeinrichtung, Regelungseinrichtung und Aktor (Durchflussregler einschließlich Antrieb) - als mechanisch voneinander getrennte Einheiten ausgebildet sind. Hierbei ermittelt eine Messeinrichtung den aktuellen Druck im Messvolumen (in der Messzelle), diese gibt dann ein Signal an die Regeleinrichtung ab, welche wiederum ein Stellsignal an einen Aktor abgibt, der den Durchströmquerschnitt variiert und somit den Druck im Messvolumen einstellt.

Vorteilhaft kann zusätzlich zur Druckregeleinrichtung am Austritt des Gases aus derselben, d.h., zwischen Druckregeleinrichtung und Entlassatmosphäre, eine Fördereinrichtung für den oder die zu analysierenden Teilströme, deren Konzentration an einem oder mehreren ausgewählten Bestandteilen gemessen wird, eingebracht sein.

Die Fördereinrichtung kann vorteilhaft eine Membranpumpe sein, wobei aber allgemein auch andere allgemein gebräuchlich Pumpen, z.B. Kolbendosierpumpen oder Schlauchpumpen oder auch andere speziell angepasste Pumpen, einsetzbar sind. Bewährt haben sich einstufige Pumpen, insbesondere Gasmembranpumpen speziell des Typs M56ex von Fa. KNF-Neuberger D-Freiburg i. Br., geeignet sind z.B. auch Gasmembranpumpen des Typs N86 und N87 der Fa. KNF-Neuberger D-Freiburg i. Br. und Faltenbalgpumpen der Typenserie P 2.2 bis P 2.6 bzw. US-P 2.6 der Fa. Bühler D-Ratingen. Es kommen auch Membranpumpen z.B. des Typs FM 1101 der Fa. Fürgut in D-Tannheim in Frage.

In dieser Ausführungsform mit Fördereinrichtung ist es möglich, bei einem stabilen Messdruck in der Messzelle des Analysengerätes oder der Analysengeräte zu arbeiten, der in im wesentlichen beliebiger Weise vom Druck in der Entlassatmosphäre abweicht. Der Messdruck kann in einer Ausführungsvariante unterhalb des Druckes der Entlassatmosphäre liegen, in einer anderen Ausführungsvariante aber auch gleich oder höher als der Druck der Entlassatmosphäre sein.

Für die Messgenauigkeit ist es vorteilhaft, bei Überdruck gegenüber dem Druck in der Entlassatmosphäre zu arbeiten, weil dadurch selbst bei geringsten Fertigungsleckagen keine Gase von außen in das Messvolumen (in die Messzelle) eindringen, die das Messergebnis verfälschen könnten. Diese Verfahrensvariante ist z.B. dann besonders zweckmäßig, wenn der Bestandteil des zu analysierenden Gasstromes, dessen Konzentration gemessen wird, Sauerstoff ist, da die das Messgerät und somit das Messvolumen umgebende Atmosphäre in der Regel sauerstoffhaltige Luft ist.

Enthält dagegen der zu analysierende Gasstrom, dessen Konzentration wenigstens eines Bestandteils gemessen wird, eine toxische Substanz, so ist eine Messung bei Unterdruck gegenüber dem Druck in der Entlassatmosphäre (häufig die natürliche Messraumumgebung) häufig vorzuziehen, um Leckagen der toxischen Substanz nach außen zu vermeiden. Die erhöhte Sicherheit geht unter Umständen jedoch zu Lasten einer begrenzten Messgenauigkeit infolge des Eindringens von Fremdgas.

Erfindungsgemäß besonders zweckmäßig wird der Druck innerhalb der Messzelle um nicht mehr als + 100 mbar vom Druck in der Entlassatmosphäre, bzw. vom Druck im zu analysierenden Gasstrom abweichen. Dieser Sachverhalt liegt darin begründet, dass der erfindungsgemäße Druckregelkreis im Sinne einer hohen Messgenauigkeit insgesamt sehr empfindlich reagieren muss. Hohe Druckdifferenzen sind einer solchermaßen erforderlichen Drucksensibilität in der Regel abträglich.

In einer weiteren vorteilhaften Ausgestaltung der erfindungsgemäßen Verfahrensweise können Teilströme von zwei oder mehr voneinander unabhängigen, unabhängig voneinander zu analysierenden Gasströmen zur Messung der jeweiligen Konzentration eines oder mehrerer ihrer Bestandteile jeweils einer entsprechenden Anzahl von parallel betriebenen Analysengeräten zugeführt und die aus den Analysengeräten austretenden Gasströme zum Zweck der Druckregelung der Messung in jedem einzelnen Analysengerät miteinander zu einem Gemischstrom vermischt nur einer einzigen Druckregeleinrichtung zugeführt und nachfolgend in die Entlassatmosphäre entlassen werden. Vorstehende Variante erweist sich insbesondere dann als vorteilhaft, wenn die unterschiedlichen Gasstrombestandteile miteinander chemisch verträglich sind.

Ein wesentlicher Vorteil dieser Verfahrensvariante liegt darin, dass die Fehlerbetrachtung für alle zu analysierenden Gasströme identisch ist, da die Konzentrationen eines oder mehrerer ausgewählter Bestandteile aus allen zu analysierenden Gasströmen unter jeweils identischen Bedingungen bestimmt wird. Darüber hinaus ergibt sich aus dem Sachverhalt, dass für alle Messungen nur eine einzige Druckregeleinrichtung erforderlich ist, auch ein Kostenvorteil.

Erfindungsgemäß vorteilhaft kann die vorbeschriebene Fördereinrichtung für den oder die Teilströme der zu analysierenden Gasströme, deren Konzentration eines oder mehrerer ausgewählter Bestandteile ermittelt wird, benutzt werden, um den oder die zu analysierenden Teilströme in den jeweiligen Gasstrom, woraus dieselben entnommen wurden, zurückzuführen.

Diese Ausführungsvariante mit Rückführung der analysierten Teilströme mittels der Fördereinrichtung in die zu analysierenden Gasströme der kontinuierlich betriebenen heterogen katalysierten Gasphasen-Partialoxidation ist insbesondere bei unerwünschten Ausbeuteverlusten oder aber auch bei Notwendigkeit einer Entsorgung spezieller Problemgehaltsstoffe besonders vorteilhaft, da für eine solche Entsorgung im Prozess (Partialoxidation und anschließende Zielproduktabtrennung) selbst aus anderen Gründen regelmäßig bereits entsprechende diesbezügliche Vorkehrungen vorhanden sind.

In einer weiteren Ausführungsform kann eine zusätzliche Verringerung der Regelabweichungen der Druckregeleinrichtung erreicht werden, indem z.B. ein konstanter Zusatzgasstrom (Trägergasstrom) nach (allgemein zwischen) der oder den Messzellen und der Druckregeleinrichtung zugeführt wird, so dass die Druckregeleinrichtung stets einen stabilen Mindestdurchfluss zur Verfügung hat. Erfindungsgemäß günstig wird man als einen solchen konstanten Zusatzstrom einen Gasstrom verwenden, der sich hinsichtlich der Partialoxidation als inert erweist (z.B. molekularer Stickstoff).

Häufig ist der ausgewählte Bestandteil oder einer der ausgewählten Bestandteile des Gasstromes, dessen Konzentration im Analysengerät gemessen wird, molekularer Sauerstoff.

Häufig kann der ausgewählte Bestandteil oder einer der ausgewählten Bestandteile des Gasstromes, dessen Konzentration im Analysengerät gemessen wird, Propylen und/oder Propan sein.

Abschließend sei erwähnt, dass das erfindungsgemäße Verfahren insbesondere auch zur Bestimmung des Sauerstoffgehalts für das in der DE-A 102005052923 beschriebene Verfahren herangezogen werden kann. Ferner eignet es sich zu Konzentrationsbestimmungen für das in der DE-A 102 32 482 beschriebene Verfahren.

Dem Vorgenannten zufolge eignet sich das erfindungsgemäße Verfahren insbesondere zur Anwendung im Rahmen einer heterogen katalysierten Partialoxidation von Propylen und/oder Propan zu Acrolein und/oder Acrylsäure.

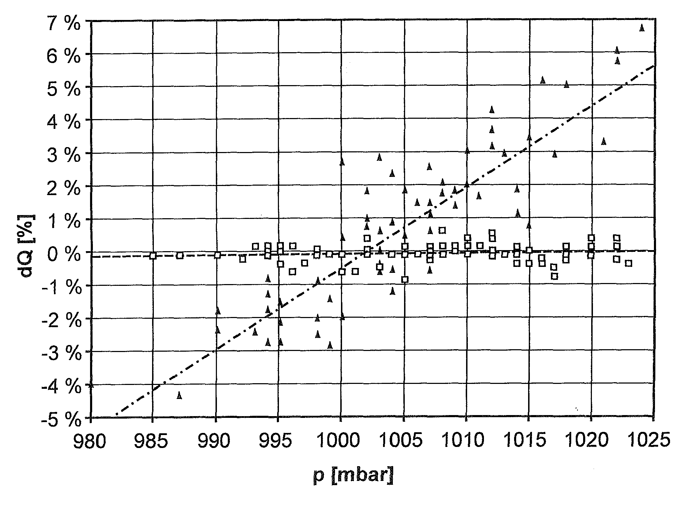

Die Erfindung wird im Folgenden anhand einer Figur sowie von Ausführungsbeispielen näher erläutert. Die einzige Figur zeigt die Ergebnisse von Messwertschwankungen der Sauerstoff-Konzentrationsmessung bei einer Luftdruckänderung in einem Analysengerät nach dem Stand der Technik, das heißt ohne Druckregeleinrichtung zum Vergleich und in einem Analysengerät mit Druckregeleinrichtung nach der Erfindung.

In der einzigen Figur ist der Umgebungsluftdruck (Entlassatmosphäre ist die natürliche äußere Umgebung im Messraum) p in mbar auf der Abszisse und die sich bei der jeweiligen Messung ergebende Abweichung in der Konzentration dQ in Prozenten vom tatsächlich eingestellten Konzentrationswert eines Referenzgases mit zertifizierter Zusammensetzung auf der Ordinate aufgeführt. Die Messabweichungen sind für ein Verfahren nach dem Stand der Technik, das heißt ohne erfindungsgemäße Regeleinrichtung des Drucks in der Messzelle des Analysengerätes durch ausgefüllte Dreiecke dargestellt. Es sind hierfür eine Vielzahl von an verschiedenen Tagen durchgeführte Messungen die rechnerisch bestimmten dimensionslosen Abweichungen der ermittelten O₂-Konzentrationswerte dQ in % vom tatsächlich eingestellten, präzise bekannten Wert der O₂-Konzentration eines vorher hergestellten Prüfgases über dem jeweils herrschenden Umgebungsluftdruck aufgetragen. Ebenso ist dies für ein erfindungsgemäßes Verfahren, mit Druckregeleinrichtung des Analysengerätes, durch hohle Quadrate dargestellt. Zu ermittelnde Spezies ist molekularer Sauerstoff, Analysengerät für die Sauerstoffkonzentration ist ein Gerät vom Typ Siemens Oxymat 5F (0 bis 10 Vol.-%). Wie aus der Figur ersichtlich, betrug die Messabweichung beim Verfahren nach dem Stand der Technik etwa ± 5 %, gegenüber nur etwa ± 0,3 % beim Verfahren nach der Erfindung. Bei Betrachtung über einen noch längeren Zeitraum würden sich aufgrund der dann noch größeren Luftdruckschwankungen aufgrund wechselnder Wetterlagen noch erheblich größere Abweichungen zeigen.

### Ausführungsbeispiele

### Vergleichsbeispiel

Aus einem kontinuierlich betriebenen Reaktor zur Partialoxidation von Propylen zu Acrylsäure wurde dem Produktgasstrom ein Teilstrom gleicher Zusammensetzung als ein auf seinen Restsauerstoffgehalt (ca. 3 Vol.-%) zu analysierender Gasstrom entnommen und einem Sauerstoff-Analysengerät Siemens Oxymat 5F zugeführt, welches in einem unklimatisierten Analysengeräteraum installiert war. Dadurch war die Messzelle im Analysengerät im Verlauf eines Jahres Schwankungen der Raumtemperatur von ca. + 3°C ausgesetzt, was einer Messabweichung im O₂-Gehalt von ca. ± 1 % (bezogen auf den Gehalt), entsprechend dem Einfluss der Temperatur auf die Dichte des zu analysierenden Gases, entspricht.

Der zu analysierende Teilgasstrom wurde aus dem Analysengerät in die Umgebungsatmosphäre entspannt. Schwankungen des Umgebungsdrucks von ± 15 mbar führten zu Messabweichungen im O₂-Gehalt von ca. + 1,5 %.

### Beispiel 1

Das Vergleichsbeispiel wurde wiederholt, wobei dem Analysengerät jedoch ein als kompakte Einheit ausgebildeter Druckregler nachgeschaltet wurde, über den der zu analysierende Teilgasstrom an die Umgebungsatmosphäre abgegeben wurde. Der Druck in der Messzelle des Analysengerätes wurde mit einer Toleranz von ± 2 mbar geregelt. Durch die Druckregelung wurde die Messabweichung im O₂-Gehalt auf rund 1/8 gegenüber dem Stand der Technik verringert.

Indem zusätzlich das Analysengerät in einem klimatisierten Raum aufgestellt und dadurch die Raumtemperatur mit einer Toleranz von ± 0,5°C konstant gehalten wurde, wurde der Messfehler aufgrund des Temperatureinflusses zusätzlich auf rund 1/6 gegenüber dem Stand der Technik vermindert.

Insgesamt konnte die Messabweichung auf unter 0,5 %, bezogen auf den O₂-Gehalt, abgesenkt werden.

### Beispiel 2

Beispiel 1 wird wiederholt, die Druckregeleinrichtung dabei jedoch als nicht-kompakte Einheit ausgebildet. Hierbei ermittelt als Messeinrichtung ein Druckmessumformer vom Typ Labom CD1020 der Fa. Labom in DE-Hude den aktuellen Druck im Messvolumen und gibt dann über eine Eingangskarte SM331 ein Signal an eine in einem Siemens PCS7-Prozessleitsystem einprogrammierte PID-Reglereinrichtung (hat einen proportionalen, einen integrierenden und einen differentiellen Wirkanteil) ab, welche wiederum über eine Ausgangskarte SM332 ein Stellsignal an einen Aktor (eine pneumatisch angetriebene Armatur Whitey SS45-S8-MM der Fa. Swagelok in Solon, Ohio, USA) abgibt, die den Durchströmquerschnitt variiert und somit den Druck im Messvolumen einstellt, wodurch die Messabweichung unter 0,3 % des O₂-Gehalts abgesenkt werden kann.

### Beispiel 3

Beispiel 2 wird mit einer weiter verbesserten Versuchsanordnung wiederholt, in der hinter dem Analysengerät und vor der Druckregeleinrichtung ein konstanter N₂-Zusatzstrom zugeführt wird. Dadurch wird eine nochmalige Verringerung der Regelabweichungen der Druckregeleinrichtung erzielt, so dass die Messabweichung nur noch 0,2 % des O₂-Gehalts beträgt.

## Patentansprüche

1. Verfahren zum sicheren Betreiben einer kontinuierlich betriebenen heterogen katalysierten Gasphasen-Partialoxidation wenigstens einer organischen Ausgangsverbindung in einem Reaktor, dessen Beschickungsgasgemischstrom neben der wenigstens einen partiell zu oxidierenden organischen Ausgangsverbindung und molekularem Sauerstoff als Oxidationsmittel wenigstens ein sich unter den Bedingungen der heterogen katalysierten Gasphasen-Partialoxidation im Wesentlichen inert verhaltendes Verdünnungsgas umfasst und der durch Zusammenführen wenigstens zweier verschiedener Ausgangsströme erzeugt wird, bei dem unter Einbezug der online-Messung der Konzentration eines oder mehrerer ausgewählter Bestandteile im Beschickungsgasgemischstrom selbst, in einem oder mehreren der den Beschickungsgasgemischstrom erzeugenden Ausgangsströme und/oder im Produktgasgemischstrom verhindert wird, dass der Reaktor mit einem explosionstechnisch oder anderweitig nicht mehr beherrschbaren Beschickungsgasgemischstrom beschickt wird, wobei zur online-Messung ein Teilstrom des jeweils zu analysierenden Gasstroms kontinuierlich in eine Messzelle eines Analysengerätes hinein- und während der durchgeführten Messung aus der Messzelle des Analysengeräts in eine Entlassatmosphäre herausgeführt wird, wobei der zu analysierende Gasstrom und/oder die Entlassatmosphäre Druckschwankungen unterliegen, das **dadurch gekennzeichnet ist, dass** der Einfluss von Schwankungen des Druckes des zu analysierenden Gasstroms und/oder der Entlassatmosphäre auf den Messdruck in der Messzelle im Analysengerät und damit auf das Messergebnis
a) rechnerisch korrigiert, und/oder
b) dadurch minimiert wird, dass der Messdruck in der Messzelle des Analysengeräts unabhängig vom Druck des zu analysierenden Gasstroms und/oder der Entlassatmosphäre mittels einer Druckregeleinrichtung konstant gestellt oder geregelt wird.

2. Verfahren nach Anspruch 1, **dadurch gekennzeichnet, dass** die Druckregeleinrichtung als einstückige kompakte Einheit ausgebildet ist.

3. Verfahren nach Anspruch 1, **dadurch gekennzeichnet, dass** die Druckregeleinrichtung als nicht kompakte Einheit ausgebildet ist.

4. Verfahren nach einem der Ansprüche 1 bis 4, **dadurch gekennzeichnet, dass** das Hineinführen des Teilstroms in die Messzelle und/oder das Herausführen des Teilstroms aus der Messzelle mit Hilfe einer Fördervorrichtung vorgenommen wird.

5. Verfahren nach Anspruch 4, **dadurch gekennzeichnet, dass** die Fördereinrichtung zwischen Druckregelungseinrichtung und Entlassatmosphäre angeordnet ist.

6. Verfahren nach Anspruch 5, **dadurch gekennzeichnet, dass** die Fördereinrichtung eine Membranpumpe ist.

7. Verfahren nach einem der Ansprüche 1 bis 6, **dadurch gekennzeichnet, dass** der Druck in der Messzelle unterhalb des Drucks in der Entlassatmosphäre liegt.

8. Verfahren nach einem der Ansprüche 1 bis 6, **dadurch gekennzeichnet, dass** der Druck in der Messzelle höher als der Druck in der Entlassatmosphäre ist.

9. Verfahren nach einem der Ansprüche 1 bis 6, **dadurch gekennzeichnet, dass** der Druck in der Messzelle dem Druck in der Entlassatmosphäre entspricht.

10. Verfahren nach einem der Ansprüche 1 bis 6, **dadurch gekennzeichnet, dass** die Abweichung des Drucks in der Messzelle sowohl vom Druck in der Entlassatmosphäre als auch vom Druck im zu analysierenden Gasstrom nicht mehr als 100 mbar beträgt.

11. Verfahren nach einem der Ansprüche 1 bis 10, **dadurch gekennzeichnet, dass** der Teilstrom des zu analysierenden Gasstroms nach seiner Herausführung aus der Messzelle in den zu analysierenden Gasstrom rückgeführt wird.

12. Verfahren nach einem der Ansprüche 1 bis 11, **dadurch gekennzeichnet, dass** zwischen Messzelle und Druckregeleinrichtung ein konstanter Zusatzstrom zugeführt wird.

13. Verfahren nach einem der Ansprüche 1 bis 12, **dadurch gekennzeichnet, dass** wenigstens einer der ausgewählten Bestandteile des zu analysierenden Gasstromes molekularer Sauerstoff ist.

14. Verfahren nach einem der Ansprüche 1 bis 12, **dadurch gekennzeichnet, dass** wenigstens einer der ausgewählten Bestandteile des zu analysierenden Gasstromes Propylen und / oder Propan ist.

15. Verfahren nach einem der Ansprüche 1 bis 14, **dadurch gekennzeichnet, dass** es in mehreren parallel betriebenen Analysengeräten zeitgleich durchgeführt und die Druckregelung für alle Messzellen der verschiedenen Analysengeräte mit nur einer gemeinsamen Druckregelung durchgeführt wird, der die zu analysierenden Teilströme aus den verschiedenen Messzellen der verschiedenen Analysengeräte als Gemisch zugeführt werden.

16. Verfahren nach einem der Ansprüche 1 bis 15, **dadurch gekennzeichnet, dass** die Entlassatmosphäre die natürliche Umgebungsatmosphäre ist.

17. Verfahren nach einem der Ansprüche 1 bis 15, **dadurch gekennzeichnet, dass** die Entlassatmosphäre nicht die natürliche Umgebungsatmosphäre ist.

18. Verfahren nach einem der Ansprüche 1 bis 17, **dadurch gekennzeichnet, dass** zusätzlich die Temperatur der Messzelle konstant geregelt wird.

## Claims

1. A method for the safe operation of a continuously operated heterogeneously catalyzed gas-phase partial oxidation of at least one organic starting compound in a reactor whose feed gas mixture stream comprises at least one diluent gas substantially inert under the conditions of the heterogeneously catalyzed gas-phase partial oxidation, in addition to the at least one organic starting compound to be partially oxidized and molecular oxygen as an oxidizing agent and is produced by combining at least two different starting streams, in which inclusion of the online measurement of the concentration of one or more selected constituents in the feed gas mixture stream itself, in one or more of the starting streams producing the feed gas mixture stream and/or in the product gas mixture stream prevents the reactor from being fed with an explosive feed gas mixture stream or a feed gas mixture stream which is otherwise uncontrollable, where, for the online measurement, a part-stream of the gas stream to be analyzed in each case is passed continuously into a measuring cell of an analytical apparatus and, while the measurement is being carried out, is passed out of the measuring cell of the analytical apparatus into a discharge atmosphere, the gas stream to be analyzed, and/or the discharge atmosphere, being subject to pressure variations, wherein the influence of variations of the pressure of the gas stream to be analyzed and/or of the discharge atmosphere on the measured pressure in the measuring cell in the analytical apparatus and hence on the result of the measurement
a) is computationally correct and/or
b) minimized by keeping constant or regulated the measured pressure in the measuring cell of the analytical apparatus independently of the pressure of the gas stream to be analyzed and/or of the discharge atmosphere by means of a pressure control apparatus.

2. The method according to claim 1, wherein the pressure control apparatus is in the form of an integral compact unit.

3. The method according to claim 1, wherein the pressure control apparatus is in the form of a non-compact unit.

4. The method according to any of claims 1 to 4, wherein the passage of the part-stream into the measuring cell and/or the discharge of the part-stream from the measuring cell is carried out with the aid of a transport apparatus.

5. The method according to claim 4, wherein the transport apparatus is arranged between pressure control apparatus and discharge atmosphere.

6. The method according to claim 5, wherein the transport apparatus is a diaphragm pump.

7. The method according to any of claims 1 to 6, wherein the pressure in the measuring cell is below the pressure in the discharge atmosphere.

8. The method according to any of claims 1 to 6, wherein the pressure in the measuring cell is higher than the pressure in the discharge atmosphere.

9. The method according to any of claims 1 to 6, wherein the pressure in the measuring cell corresponds to the pressure in the discharge atmosphere.

10. The method according to any of claims 1 to 6, wherein the deviation of the pressure in the measuring cell both from the pressure in the discharge atmosphere and from the pressure in the gas stream to be analyzed is not more than 100 mbar.

11. The method according to any of claims 1 to 10, wherein, after it has been discharged from the measuring cell, the part-stream of the gas stream to be analyzed is recycled into the gas stream to be analyzed.

12. The method according to any of claims 1 to 11 wherein a constant additional stream is fed in between measuring cell and pressure control apparatus.

13. The method according to any of claims 1 to 12, wherein at least one of the selected constituents of the gas stream to be analyzed is molecular oxygen.

14. The method according to any of claims 1 to 12, wherein at least one of the selected constituents of the gas stream to be analyzed is propylene and/or propane.

15. The method according to any of claims 1 to 14, wherein it is carried out in a plurality of analytical apparatuses operated in parallel, and the pressure control for all measuring cells of the various analytical apparatuses is carried out with only one common pressure control, into which the part-streams to be analyzed from the various measuring cells of the various analytical apparatuses are fed as a mixture.

16. The method according to any of claims 1 to 15, wherein the discharge atmosphere is the natural ambient atmosphere.

17. The method according to any of claims 1 to 15, wherein the discharge atmosphere is not the natural ambient atmosphere.

18. The method according to any of claims 1 to 17, wherein the temperature of the measuring cells is additionally kept constant.

## Revendications

1. Procédé d'exploitation sûre d'une oxydation partielle en phase gazeuse sous catalyse hétérogène exploitée en continu d'au moins un composé organique de départ dans un réacteur, dont le courant de mélange gazeux d'alimentation comprend, en plus dudit au moins un composé organique de départ à oxyder partiellement et d'oxygène moléculaire en tant qu'oxydant, au moins un gaz diluant essentiellement inerte dans les conditions de l'oxydation partielle en phase gazeuse sous catalyse hétérogène et qui est formé par réunion d'au moins deux courants de départ différents, selon lequel, en prenant en compte la mesure en ligne de la concentration d'un ou de plusieurs constituants choisis dans le courant de mélange gazeux d'alimentation lui-même, dans un ou plusieurs des courants de départ formant le courant de mélange gazeux d'alimentation et/ou dans le courant de mélange gazeux de produits, l'alimentation du réacteur avec un courant de mélange gazeux d'alimentation explosif ou autrement incontrôlable est évitée, un courant partiel du courant gazeux à analyser respectif étant introduit en continu dans une cellule de mesure d'un appareil d'analyse et déchargé de la cellule de mesure de l'appareil d'analyse dans une atmosphère de déchargement pendant la mesure réalisée pour la mesure en ligne, le courant gazeux à analyser et/ou l'atmosphère de déchargement étant soumis à des fluctuations de pression, qui est **caractérisé en ce que** l'effet des fluctuations de pression du courant gazeux à analyser et/ou de l'atmosphère de déchargement sur la pression de mesure dans la cellule de mesure dans l'appareil d'analyse et par conséquent sur le résultat de mesure
a) est corrigé par calcul et/ou
b) est minimisé en maintenant constante ou en régulant la pression de mesure dans la cellule de mesure de l'appareil d'analyse indépendamment de la pression du courant gazeux à analyser et/ou de l'atmosphère de déchargement au moyen d'un dispositif de régulation de la pression.

2. Procédé selon la revendication 1, **caractérisé en ce que** le dispositif de régulation de la pression est configuré sous la forme d'une unité compacte en un seul tenant.

3. Procédé selon la revendication 1, **caractérisé en ce que** le dispositif de régulation de la pression est configuré sous la forme d'une unité non compacte.

4. Procédé selon l'une quelconque des revendications 1 à 4, **caractérisé en ce que** l'introduction du courant partiel dans la cellule de mesure et/ou le déchargement du courant partiel de la cellule de mesure sont réalisés à l'aide d'un dispositif de transport.

5. Procédé selon la revendication 4, **caractérisé en ce que** le dispositif de transport est agencé entre le dispositif de régulation de la pression et l'atmosphère de déchargement.

6. Procédé selon la revendication 5, **caractérisé en ce que** le dispositif de transport est une pompe à membrane.

7. Procédé selon l'une quelconque des revendications 1 à 6, **caractérisé en ce que** la pression dans la cellule de mesure est inférieure à la pression dans l'atmosphère de déchargement.

8. Procédé selon l'une quelconque des revendications 1 à 6, **caractérisé en ce que** la pression dans la cellule de mesure est supérieure à la pression dans l'atmosphère de déchargement.

9. Procédé selon l'une quelconque des revendications 1 à 6, **caractérisé en ce que** la pression dans la cellule de mesure correspond à la pression dans l'atmosphère de déchargement.

10. Procédé selon l'une quelconque des revendications 1 à 6, **caractérisé en ce que** la déviation de la pression dans la cellule de mesure aussi bien par rapport à la pression dans l'atmosphère de déchargement que par rapport à la pression dans le courant gazeux à analyser n'est pas supérieure à 100 mbar.

11. Procédé selon l'une quelconque des revendications 1 à 10, **caractérisé en ce que** le courant partiel du courant gazeux à analyser est recyclé dans le courant gazeux à analyser après son déchargement de la cellule de mesure.

12. Procédé selon l'une quelconque des revendications 1 à 11, **caractérisé en ce qu'**un courant d'ajout constant est introduit entre la cellule de mesure et le dispositif de régulation de la pression.

13. Procédé selon l'une quelconque des revendications 1 à 12, **caractérisé en ce qu'**au moins un des constituants choisis du courant gazeux à analyser est l'oxygène moléculaire.

14. Procédé selon l'une quelconque des revendications 1 à 12, **caractérisé en ce qu'**au moins un des constituants choisis du courant gazeux à analyser est le propylène et/ou le propane.

15. Procédé selon l'une quelconque des revendications 1 à 14, **caractérisé en ce qu'**il est réalisé simultanément dans plusieurs appareils d'analyse exploités en parallèle et la régulation de la pression pour toutes les cellules de mesure des différents appareils d'analyse est réalisée avec uniquement une régulation de la pression commune, dans laquelle les courants partiels à analyser provenant des différentes cellules de mesure des différents appareils d'analyse sont introduits en mélange.

16. Procédé selon l'une quelconque des revendications 1 à 15, **caractérisé en ce que** l'atmosphère de déchargement est l'atmosphère ambiante naturelle.

17. Procédé selon l'une quelconque des revendications 1 à 15, **caractérisé en ce que** l'atmosphère de déchargement n'est pas l'atmosphère ambiante naturelle.

18. Procédé selon l'une quelconque des revendications 1 à 17, **caractérisé en ce que** la température de la cellule de mesure est en outre régulée pour être constante.
